Europäisches Patentamt

European Patent Office

Office européen des brevets

(11) Veröffentlichungsnummer: **0 151 278 B1**

## (12) EUROPÄISCHE PATENTSCHRIFT

(45) Veröffentlichungstag der Patentschrift: **13.03.91**

(51) Int. Cl.⁵: **G01N 31/22**

(21) Anmeldenummer: **84115253.1**

(22) Anmeldetag: **12.12.84**

Die Akte enthält technische Angaben, die nach
dem Eingang der Anmeldung eingereicht wurden
und die nicht in dieser Patentschrift enthalten sind.

(54) **Lösungsmittelgemisch zur Bestimmung des Säuregehalts von in mit Dytel-Freongasen betriebenen Kühlanlagen verwendetem Öl.**

(30) Priorität: **02.02.84 HU 44084**

(43) Veröffentlichungstag der Anmeldung:
**14.08.85 Patentblatt 85/33**

(45) Bekanntmachung des Hinweises auf die
Patenterteilung:
**13.03.91 Patentblatt 91/11**

(84) Benannte Vertragsstaaten:
**AT BE CH DE FR GB IT LI LU NL SE**

(56) Entgegenhaltungen:
**DE-A- 3 401 258        FR-A- 1 392 494**
**GB-A- 963 794          US-A- 2 770 530**
**US-A- 3 259 463        US-A- 3 580 704**
**US-A- 3 811 837        US-A- 4 288 402**

**PATENT ABSTRACTS OF JAPAN, Band 6, Nr.**
**166 (P-138) [1044], 31. August 1982 & JP-**
**A-57-82 763**

(73) Patentinhaber: **Hütogépgyár**
**Postfach 64**
**H-5101 Jászberény(HU)**

(72) Erfinder: **Sebestyen, Györgyné, Dipl.-Ing.**
**Erdöalja u. 91**
**H-1037 Budapest(HU)**

(74) Vertreter: **Brommer, Hans Joachim, Dr.-Ing. et**
**al**
**Patentanwälte Dipl.-Ing. R. Lemcke Dr.-Ing.**
**H.J. Brommer Bismarckstrasse 16 Postfach**
**4026**
**W-7500 Karlsruhe 1(DE)**

## Beschreibung

Die Erfindung betrifft ein Lösungsmittelgemisch zur Bestimmung des Säuregehalts von in mit Dytel-® Freongasen betriebenen Kühlanlagen verwendetem Öl.

Es ist eine wohlbekannte Tatsache, daß der Säuregehalt des in Kühlanlagen verwendeten Öls aus dem Standpunkt der Betriebssicherheit der Anlage von äußerster Bedeutung ist. Aus diesem Grund muß das in die Anlage einzufüllende Kühlanlagenöl qualitativ, auf mehrere Parameter, u.a. auf Säure geprüft werden.

Um die komplizierte Prüfung vermeiden zu können und daß die Kühlanlagenmonteure nicht gezwungen seien während der Montage und des Betriebs der Kühlanlage das Öl ins Laboratorium zwecks Überprüfung zu schicken, hat man zahlreiche Prüfungsmethoden entwickelt.

Aus denen ist der s.g. Sporlan ACID Test der bekannteste, der in den US-PS 3 811 837 ausführlich beschrieben ist.

Die zitierten Verfahren sind zur schnellen Säurebestimmung der Öle geeignet, aber entweder ist der Bedarf an Lösungsmittel zu hoch, oder ändert sich der Wirkungswert der Lösungen infolge der Verpackung, so ist ihre Lagerungszeit bedeutend beschränkt.

Eine andere Forschungstendenz wird durch das Verfahren repräsentiert, im Laufe dessen das zu prüfende Öl in die Lösung von 45 Vol. abs. Ethanol, 45 Vol .% Xylol von analytischer Reinheit 10 Vol .% 0,01 n/mit l-Faktor/ethanolhaltige KOH Lösung, sowie 0,04 % Bromthymolblau-Indikator eingeführt wird, wobei die Farbänderung des Gemisches registriert wird; je Ölmuster wird frisches Kühlanlagenöl , bei mit Freon® betriebenen Kühlanlagen werden Frischöl und Altöl verwendet.

Das bei dem oben erwähnten Verfahren zur Verwendung kommende Lösungsmittel erleichtert wesentlich die Bestimmung des Säuregehalts der bei mit Freon betriebenen Kühlanlagen verwendeten Öle und ist dazu geeignet die Bestimmung in einer kurzen Zeit - praktisch in einigen Minuten - durchführen zu können, der Bedarf an Lösungsmittel ist minimal , qualifiziertes Personal ist nicht beansprucht, dabei ist das Lösungsmittelgemisch so verpackt: daß das Lösungsmittelgemisch unbegrenzt gelagert werden kann.

Ein derartiges Verfahren ist in der ungarischen Patentanmeldung 151/83 beschrieben, das aber keinesfalls zur Bestimmung des Säuregehalts des stark rot gefärbten Öls von Kühlanlagen, die mit rotem Farbstoff versehenen Freongasen betrieben sind, geeignet ist.

Außerdem ist durch Patent Abstracts of Japan, Band 6, 166 (P-138) (1044), 31. August 1982, ein Lösungsmittelgemisch zur Bestimmung des Säuregehaltes in Ölen bekannt, daß aus Toluol und Alkohol zum Lösen des zu untersuchenden Öls, aus KOH zur Festlegung des maximal zulässigen Säuregehaltes und aus Bromthymol - blau als pH-Farbindikator besteht. Auch dieses Gemisch ist jedoch nicht für Öle geeignet, die mit Dytel®-Rotfarbstoff versetzt sind. Außerdem finden sich in der Entgegenhaltung keinerlei numerische Angaben über die Mengenanteile der verschiedenen Komponenten.

Bei den mit dem s.g. Dytel Rotfarbstoff behandelten Freongasen betriebenen Kühlanlagen sind nämlich die Kühlanlagenöle stark rot gefärbt. Aus diesem Grunde sind weder die ausländischen, noch die einheimischen Prüfungsmethoden zur Säurebestimmung von Dytel-Farbstoff enthaltenden Ölen geeignet, da die rote Farbe die Farbe der Säureindikatoren überdeckt. Demnach muß zur Säurebestimmung der rote Dytel-Farbstoff in eine separate Phase überführt werden.

Der Erfindung wurde das Ziel gesetzt ein Lösungsmittelgemisch zur Bestimmung des Säuregehalts von in mit Dytel-Freo$_n$® gasen betriebenen Kühlanlagen verwendetem Öl zu entwickeln, das zur Bestimmung des Säuregehalts von mit Dytel-Freonfarbstoff versehenen Kühlanlagenölen geeignet ist.

Erfindungsgemäß wird das gesetzte Ziel mit einem Lösungsmittelgemisch erreicht, das dadurch gekennzeichnet werden kann, daß es 45 ml abs. Ethanol, 45 ml Xylol, 10 ml 0,01n alkoholische KOH Lösung (mit 1-Faktor) und 0,01 - 0,1 Massen-% wässeriger Bromthymolblau-Indikator und destilliertes Wasser enthält.

Das erfindungsgemäße Lösungsmittelgemisch wird folgenderweise verwendet:

Aus dem Gemisch werden 2,4 ml in eine durchsichtige Glasampulle mit einem Rauminhalt von 5 cm³ eingegeben, wonach 1 ml kolendioxidfreies destilliertes Wasser zugemischt wird, danach wird die Ampulle luftdicht abgelötet.

Beabsichtigen wir nun den Säuregehalt zu überprüfen, geben wir zu der Lösung in der geöffneten Ampulle mit einem Tropfröhrchen 12 Tropfen des zu prüfenden, mit Dytel®-Farbstoff verunreinigten Kühlanlagenöls.

Nachdem das Gemisch gründlich geschüttelt worden ist, warten wir 5-6 Minuten bis die beiden Phasen sich trennen, wonach der Säuregehalt des Öls bestimmt werden kann.

Unsere Versuche haben es gezeigt, daß im Laufe der Phasentrennung die gesamte Säuremenge aus dem Öl in die untere Phase gelangt, während das mit dem roten Farbstoff verunreinigte Lösungsmittel sich in der oberen Phase befindet.

Wenn die Farbe der unteren Phase blau bleibt, so liegt das geprüfte Öl unter dem zulässigen Säurepegel (0,05 mg KOH/g Öl).

Wenn die untere Phase eine grünlichblau Farbe aufweist, so ist der Säurepegel eben am Grenzwert.

Wenn aber die untere Phase gelb ist, so ist das geprüfte Öl übermäßig säurehaltig, das heißt, der Säuregehalt liegt über dem zulässigen Grenzwert.

1. Beispiel

Die Zusammensetzung des Lösungsmittelgemisches für 100 ml Lösung:
45 ml abs. Ethanol von analytischer Reinheit
10 ml 0,01 n alkoholhaltige KOH-Lösung mit 1-Faktor 0,04 Massen-% wässeriger Bromthymolblau-Indikator (50-60 Tropfen)

Aus diesem Gemisch nimmt man 2,4 ml und gibt man 1 ml $CO_2$-freies destilliertes Wasser dazu.

## Ansprüche

1. Lösungsmittelgemisch zur Bestimmung des Säuregehaltes von Öl, das in mit Dytel®-Freongasen betriebenen Kühlanlagen verwendet wird, wobei dieses Gemisch 45 ml abs. Ethanol , 45 ml Xylol , 10 ml 0,01 n alkoholhaltige KOH Lösung, 0,01 - 0,1 Massen-% wässerigen Bromthymolblau-Indikator und destilliertes Wasser enthält.

## Claims

1. A solvent mixture for determining the acid content of oil which is destined for use in cooling plants operated with Dytel® - Freon gases, wherein this mixture contains 45 ml absolute ethanol, 45 ml xylene, 10 ml of 0.01 N alcohol-containing KOH solution, 0.01 - 0.1 weight percent aqueous bromothymol blue indicator and distilled water.

## Revendications

1. Mélange de solvants pour la détermination de la teneur en acide d'une huile qui est utilisée dans des installations de refroidissement fonctionnant aux gaz Dytel®-Fréon®, ce mélange contenant 45 ml d'éthanol absolu, 45 ml de xylène, 10 ml de solution alcoolique de KOH 0,01 n, du bleu de bromothymol indicateur aqueux à 0,01-0,1% en masse et de l'eau distillée.